# EUROPEAN PATENT SPECIFICATION

(11) **EP 2 285 978 B1**
(45) Date of publication and mention of the grant of the patent: **08.01.2014**
(21) Application number: 09749920.6
(22) Date of filing: 22.05.2009
(51) Int. Cl.: C12Q 1/68

(54) **IMPROVED NUCLEIC ACID AMPLIFICATION WITH SINGLE STRAND DNA BINDING PROTEIN**
VERBESSERTE NUKLEINSÄUREAMPLIFIKATION MIT EINZELSTRANG-DNA-BINDENDEM PROTEIN
AMPLIFICATION AMÉLIORÉE D ACIDE NUCLÉIQUE AVEC UNE PROTÉINE DE LIAISON À L ADN SIMPLE BRIN

(30) Priority: 22.05.2008 US 55167 P
(43) Date of publication of application: 23.02.2011
(73) Proprietor: GE Healthcare Bio-Sciences Corp., Piscataway, N.J. 08855 (US)
(72) Inventor: KUMAR, Gyanendra, Piscataway, New Jersey 08855 (US); GARNOVA, Elena, White Plains, New York 10601 (US); HAMILTON, Scott, Phillipsburg, New Jersey 08855 (US); CHERNAYA, Galina, Princeton, New Jersey 08540 (US)
(74) Representative: Aldenbäck, Ulla Christina
(86) International application number: PCT/EP2009/056235
(87) International publication number: WO 2009/141430

(56) References cited:
- EP-A- 1 754 716
- EP-A1- 1 916 311
- US-A1- 2005 277 146
- INOUE JIN ET AL: "Improvements of rolling circle amplification (RCA) efficiency and accuracy using Thermus thermophilus SSB mutant protein." NUCLEIC ACIDS RESEARCH 2006, vol. 34, no. 9, 2006, page e69, XP002541058 ISSN: 1362-4962
- CANCEILL D ET AL: "Replication slippage of different DNA polymerases is inversely related to their strand displacement efficiency." THE JOURNAL OF BIOLOGICAL CHEMISTRY 24 SEP 1999, vol. 274, no. 39, 24 September 1999 (1999-09-24), pages 27481-27490, XP002541059 ISSN: 0021-9258
- SIMISON W B ET AL: "Rolling circle amplification of metazoan mitochondrial genomes" MOLECULAR PHYLOGENETICS AND EVOLUTION, ORLANDO, FL, US, vol. 39, no. 2, 1 May 2006 (2006-05-01), pages 562-567, XP024907037 ISSN: 1055-7903 [retrieved on 2006-05-01]
- DEAN F B ET AL: "Rapid amplification of plasmid and phage DNA using Phi29 DNA polymerase and multiply-primed rolling circle amplification" GENOME RESEARCH, COLD SPRING HARBOR LABORATORY PRESS, WOODBURY, NY, US, vol. 11, no. 6, 1 June 2001 (2001-06-01), pages 1095-1099, XP002223174 ISSN: 1088-9051
- MIKAWA TSUTOMU ET AL: "Single-stranded DNA binding protein facilitates specific enrichment of circular DNA molecules using rolling circle amplification." ANALYTICAL BIOCHEMISTRY 15 AUG 2009, vol. 391, no. 2, 12 May 2009 (2009-05-12), pages 81-84, XP002541060 ISSN: 1096-0309
- CHEN FENG ET AL: "Genomic sequence and evolution of marine cyanophage P60: a new insight on lytic and lysogenic phages." APPLIED AND ENVIRONMENTAL MICROBIOLOGY MAY 2002, vol. 68, no. 5, May 2002 (2002-05), pages 2589-2594, XP002541061 ISSN: 0099-2240

## Description

### Field of the Invention

The methods disclosed relate to improved methods of DNA amplification to provide desired products with higher purity.

### Background of the Invention

Several useful methods have been developed that permit amplification of nucleic acids. Most were designed around the amplification of selected DNA targets and/or probes, including the polymerase chain reaction (PCR), ligase chain reaction (LCR), self-sustained sequence replication (3SR), nucleic acid sequence based amplification (NASBA), strand displacement amplification (SDA), and amplification with Qβ replicase (Birkenmeyer and Mushahwar, J. Virological Methods, 35:117-126 (1991); Landegren, Trends Genetics, 9:199-202 (1993)). In addition, several methods have been employed to amplify circular DNA molecules such as plasmids or DNA from bacteriophage such as M13. One application has been propagation of these molecules in suitable host bacteria such as strains of *E. coli,* followed by isolation of the DNA by well-established protocols (Sambrook, J., Fritsch, E. F., and Maniatis, T. Molecular Cloning, A Laboratory Manual, 1989, Cold Spring Harbor Laboratory Press, Cold Spring Harbor, N.Y.). PCR has also been a frequently used method to amplify defined sequences in DNA targets such as plasmids and DNA from bacteriophage such as M13. Some of these methods suffer from being laborious, expensive, time-consuming, inefficient, and lacking in sensitivity. They may also require specific knowledge about the sequences to be amplified.

As an improvement on these methods, linear rolling circle amplification (LRCA) uses a primer annealed to a circular target DNA molecule and DNA polymerase is added. The amplification target circle (ATC) forms a template on which new DNA is made, thereby extending the primer sequence as a continuous sequence of repeated sequences complementary to the circle but generating only about several thousand copies per hour. An improvement on LRCA is the use of exponential RCA (ERCA), with additional primers that anneal to the replicated complementary sequences to provide new centers of amplification, thereby providing exponential kinetics and increased amplification. Exponential rolling circle amplification (ERCA) employs a cascade of strand displacement reactions, also referred to as HRCA (Lizardi, P. M. et al. Nature Genetics, 19, 225-231 (1998)).

European patent application EP 1 916 311 A1 discloses a method for amplifying circular (e.g. mitochondrial) DNA from a mixture comprising circular DNA and linear genomic DNA by using rolling circle amplification and incubating the mixture with a reaction system comprising Phi29 DNA polymerase.

In US Patent No. 6,323,009, a means of amplifying target DNA molecules is introduced. This method is of value because such amplified DNA is frequently used in subsequent methods including DNA sequencing, cloning, mapping, genotyping, generation of probes for hybridization experiments, and diagnostic identification.

The methods of the US 6,323,009 patent (referred to herein as Multiple Primed Amplification--MPA) improve the sensitivity of linear rolling circle amplification by using multiple primers for the amplification of individual target circles. The MPA method has the advantage of generating multiple tandem-sequence DNA (TS-DNA) copies from each circular target DNA molecule. In addition, MPA has the advantages that in some cases the sequence of the circular target DNA molecule may be unknown while the circular target DNA molecule may be single-stranded (ssDNA) or double-stranded (dsDNA or duplex DNA). Another advantage of the MPA method is that the amplification of single-stranded or double-stranded circular target DNA molecules may be carried out isothermally and/or at ambient temperatures. Other advantages include being highly useful in new applications of rolling circle amplification, low cost, sensitivity to low concentration of target circle, flexibility, especially in the use of detection reagents, and low risk of contamination.

The MPA method can improve on the yield of amplified product DNA by using multiple primers that are resistant to degradation by exonuclease activity that may be present in the reaction. This has the advantage of permitting the primers to persist in reactions that contain an exonuclease activity and that may be carried out for long incubation periods. The persistence of primers allows new priming events to occur for the entire incubation time of the reaction, which is one of the hallmarks of ERCA and has the advantage of increasing the yield of amplified DNA.

The MPA method allows for the first time *"in vitro* cloning", i.e. without the need for cloning into an organism, of known or unknown target DNAs enclosed in circles. A padlock probe may be used to copy the target sequence into a circle by the gap fill-in method (Lizardi, P. M. et al. Nature Genetics, 19,225-231 (1998)). Alternatively, target sequences can be copied or inserted into circular ssDNA or dsDNA by many other commonly used methods. The MPA amplification overcomes the need to generate amplified yields of the DNA by cloning in organisms such as bacterial host cells.

The MPA method is an improvement over LRCA in allowing increased rate of synthesis and yield. This results from the multiple primer sites for DNA polymerase extension. Random primer MPA also has the benefit of generating double stranded products. This is because the linear ssDNA products generated by copying of the circular template will themselves be converted to duplex form by random priming of DNA synthesis. Double stranded DNA product is advantageous in allowing for DNA sequencing of either strand and for restriction endonuclease digestion and other methods used in cloning, labeling, and detection.

It is also expected that strand-displacement DNA synthesis may occur during the MPA method resulting in an exponential amplification. This is an improvement over conventional ERCA, also termed HRCA (Lizardi et al. (1998)) in allowing for the ability to exponentially amplify very large linear or circular DNA targets. The amplification of large circular DNA, including bacterial artificial chromosomes (BACs), has been reduced to practice using the MPA method.

Methods have published for whole genome amplification using degenerate primers (Cheung, V. G. and Nelson, S. F. Proc. Natl. Acad. Sci. USA, 93, 14676-14679 (1996) and random primers (Zhang, L. et al., Proc. Natl. Acad. Sci. USA, 89, 5847-5851 (1992) where a subset of a complex mixture of targets such as genomic DNA is amplified. Reduction of complexity is an objective of these methods. A further advantage of the MPA method is that it amplifies DNA target molecules without the need for "subsetting", or reducing the complexity of the DNA target.

The MPA method rapidly amplifies every sequence within the sample of DNA used with it, the double-stranded product has all the same sequences as the original sample. Except for the fact that it contains tandemly-repeated copies of the DNA with numerous initiation (priming) sites, the physical properties of the product DNA are much like those of the starting template.

It has been found that when a mixture of long, linear and circular DNAs are provided for MPA, sequences within both forms are amplified. However, the majority of cloned DNAs grown in bacterial and other hosts are circular such as the DNA found in plasmids, bacterial artificial chromosomes (BACs), fosmids, cosmids, certain bacteriophage clones such as M13 clones and others. It is common to seek to isolate the clone sequences separate from those of the host cells for further analysis. It should be noted that host cell chromosomes are much larger than BAC clones and are nearly always isolated as broken linear fragments of the circular chromosome.

Accordingly, there is a need for amplification methods that lack the limitations of PCR and which favor amplification of circular forms of DNA over long, linear forms. These concerns are addressed in greater detail below.

### Summary of the Invention

New methods of nucleic acid amplification are disclosed in which the presence of single strand DNA binding protein (SSB) improves the purity and yield of desired amplification products. The methods are applicable to circular DNA molecules, especially mitochondrial DNA.

### Detailed Description of the Invention

Phi 29 DNA polymerase has proved useful in several amplification methods. These include Rolling Circle Amplification (RCA) and Multiple Displacement Amplification (MDA). RCA is particularly useful for amplifying circular DNA molecules, e.g. plasmids and MDA can be used to amplify linear DNA especially genomic DNA. However, if the starting material contains both circular DNA and linear DNA molecules both RCA and MDA will amplify both types of molecules albeit one might be to a lesser extent. Nevertheless, at the end of the amplification reaction, the product will consist of both circular and linear DNA molecules.

It has been surprisingly found that inclusion of SSB in reaction mixtures comprising both circular and genomic linear DNA molecules leads to a much increased yield of circular DNA amplified products using RCA. This has been particularly useful for the amplification of circular mitochondrial DNA in samples which also contain genomic chromosomal DNA. This sample can be obtained from a cell extract. Other circular DNA molecules e.g. viral DNA have been selectively amplified over genomic chromosomal DNA when SSB is present in the amplification reaction mixture. It was found that when a starting sample of DNA containing both circular mitochondrial DNA and linear genomic DNA was amplified using GEHC TempliPhi kit comprising Phi 29 DNA polymerase, random hexamer primers, dNTPs and buffer without added SSB, there was no enrichment of mitochondrial DNA. However the content of mitochondrial DNA increased as much as 70-80 fold compared with genomic chromosomal DNA when 100ng of either *E. coli* SSB or Tth255-SSB from Thermus thermophilus was present in the reaction mixture. The SSB binding protein from T7 (T7 Gp2 3:1) was also shown to specifically help to increase the content of mitochondrial circular DNA in the amplified product. Increased mitochondrial circular DNA was present in the amplified product when SSB was used in the range of 50-1000ng per reaction mixture. The reactions were incubated at 30°C for 16 hours. Preferably, the starting DNA sample should not be heat denatured to reduce circular DNA nicking, which is important for RCA of circular target DNA.

The product of the amplified reaction can be sequenced directly using specific forward and reverse primers for mitochondrial DNA. The ability to produce mitochondrial DNA more easily in a simple one tub sample preparation is important to determine biomarkers based on mutation in mitochondrial genomes for cancer, inherited and metabolic diseases.

It was also observed that if the amplification reaction was performed in the presence of SSB and mitochondrial sequence specific primers in place of random hexamers then up to 1500 fold enrichment of mitochondrial DNA was observed. Tth255-SSB gave the best enrichment whilst *E. coli* SSB produced only about half that enrichment.

Amplification Protocol: 10 ng human gDNA which contains approximately 10-20 pg mtDNA (0.1-0.2%) was mixed with 9 µl sample buffer containing 20 mM Tris-HCl pH 8.0 and 3µM each specific primers. To it were added 9µl TempliPhi 100 reaction buffer and 10 ng Phi29 DNA polymerase and 100 ng *E. coli* SSB or 175 ng Tth255-SSB. The amplification was carried out at 30°C for 8 hrs (when *E. coli* SSB is used) or 16 hrs (when Tth255-SSB is used). The amplification is stopped by heat inactivation of the enzyme for 20 min at 65°C. Where appropriate the specific primers can be replaced by an appropriate amount of random hexamer primers.

Whilst the results reported use Phi29 DNA polymerase, similar results would be expected using related DNA polymerases e.g. Phi15 DNA polymerase (WO 2006/073892). These enzymes are defined as Phi29 type DNA polymerase. The DNA polymerases have the property of producing long amplification products and have strand displacement activity. Any DNA polymerase with these properties can be used in the disclosed methods and are intended to be encompassed within this disclosure.

The methods described can also be applied to the investigation of circular viral genomes. This can include the discovery of new DNA viruses for which partial sequence may be known such as conserved motifs for the family.

## Claims

1. A method for preferentially amplifying circular DNA from a mixture comprising circular DNA and genomic linear DNA by incubating the mixture with reaction system comprising Phi29 DNA polymerase and a single strand DNA binding protein,
where the circular nucleic acid amplification is Rolling Circle Amplification.

2. The method of claim 1, where the single strand DNA binding protein is *E.coli* SSB, or Tth225-SSB from Thermus thermophilus, or T7 Gp2 3:1 from T7.

3. The method of claim 2, wherein the circular DNA is mitochondrial DNA.

## Patentansprüche

1. Verfahren zur bevorzugten Amplifikation von zirkulärer DNA aus einer Mischung, die zirkuläre DNA und genomische, lineare DNA enthält, durch Inkubieren der Mischung mit einem Reaktionssystem, das Phi29-DNA-Polymerase und ein Einzelstrang-DNA bindendes Protein enthält, wobei die Amplifikation der zirkulären Nukleinsäure eine Rolling-Circle-Amplifikation ist.

2. Verfahren nach Anspruch 1, wobei das Einzelstrang-DNA bindende Protein *E*. *coli* SSB oder Tth225-SSB aus Thermus thermophilus oder T7 Gp2 3:1 aus T7 ist.

3. Verfahren nach Anspruch 2, wobei die zirkuläre DNA mitochondriale DNA ist.

## Revendications

1. Procédé pour amplifier de manière préférentielle un ADN circulaire à partir d'un mélange comprenant un ADN circulaire et un ADN linéaire génomique en faisant incuber le mélange avec un système de réaction comprenant l'ADN polymérase Phi29 et une protéine de liaison à l'ADN simple brin,
où l'amplification de l'acide nucléique circulaire est une amplification par cercle roulant.

2. Procédé selon la revendication 1, où la protéine de liaison à l'ADN simple brin est la protéine de liaison à l'ADN simple brin de l'*E. Coli* ou la protéine de liaison à l'ADN simple brin Tth225 du Thermus thermophilus, ou le T7 Gp2 3:1 du T7.

3. Procédé selon la revendication 2, dans lequel l'ADN circulaire est un ADN mitochondrial.
